# EUROPEAN PATENT APPLICATION

(11) **EP 1 547 610 A1**
(43) Date of publication of application: **29.06.2005**
(21) Application number: 03079161.0
(22) Date of filing: 23.12.2003
(51) Int. Cl.: A61K 39/00

(54) **Immunotherapy for food allergy by reduced and alkylated food allergens**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Koppelman, Stefan Johan, 3731 EG De Bilt (NL); Penninks, Andreas Hendrikus, 3904 BV Veenendaal (NL); Knippels, Léon Mathieu Johannes, 3523 VJ Utrecht (NL)
(74) Representative: Prins, Adrianus Willem, Mr. Ir.

(57) **Abstract**

The invention relates to a treatment of human individuals suffering from food allergy. The treatment involves a specifically modified food allergen and is based on immunotherapy. The modified allergen is derived from a dietary protein, preferably a tree nut protein and is modified by reduction and alkylation.

## Description

The invention relates to a treatment of human individuals suffering from food allergy.

Humans rather frequently suffer from more or less severe allergic reactions after consumption of dietary proteins. The prevalence of food allergy is approximately 1-2% in adults and 6-8% in children. Food allergy is mainly associated with a limited range of foodstuffs, mainly peanuts, tree nuts, hen's eggs, cow's milk, wheat (gluten), soybeans, fish and shellfish.

The occurrence of allergic reactions is associated with a reaction of an individual's immune system to exposure of a particular allergen. In an individual having the tendency to develop an allergy to a particular allergen, first time exposure normally does not give rise to any allergic reactions. The allergen is taken up by an antigen presenting cell (APC), such as a macrophage or dendritic cell, which degrades the allergen. Fragments of the allergen are presented to CD4+ T-cells, which may respond in essentially two different ways. T-cells secrete cytokines which have effects on other cells of the immune system, most notably B-cells. They are subdivided into two categories. The first category contains Thelper1-cells, secreting a.o. interleukin-2 (IL-2) and interferon-γ (IFN-γ). The presence of IFN-γ will induce B-cells to produce specific subclasses of IgG antibodies. The second category contains Thelper2-cells. These secrete different cytokines such as IL-4, IL-5 and IL-13 . Production of IL-4 and IL-13 are necessary for the initiation and maintenance of IgE antibodies produced by B cells. These IgE antibodies will have specificity for the allergen.

Upon every additional exposure of the individual to the particular allergen, said allergen will bind to the available IgE antibodies, particularly to those bound to the surface of mast cells or basophils. As allergens typically have several sites that can bind to the IgE antibodies, those antibodies in effect become crosslinked. The result of the crosslinking of the surface-bound IgE antibodies is that the mast cells and basophils degranulate and release mediators like histamines that trigger allergic reactions.

To date, no effective treatment of food allergies is available. Most allergic individuals adapt to their situation and avoid intake of foodstuffs to which they are allergic. Therapies involving drugs, such as antihistamines, decongestants, or steroids, are available but only combat the symptoms of an allergic reaction. They do not prevent that future exposures to the allergen cause new allergenic reactions.

In the past, it has been proposed to treat allergies on the basis of immunotherapy. Such treatment involves repeated injections of allergen extracts over a long period to desensitize a patient to the allergen. This therapy is, however, very time consuming, usually involving years of treatment, and frequently fails to achieve the goal of desensitizing the patient to the allergen. Moreover, particularly for food allergies, it is not a safe treatment. With many food allergies, allergic reactions are associated with a significant risk of anaphylaxis, which is a systemic and potentially lethal type of allergic reaction.

Accordingly, there is a need for an efficacious treatment of allergies, in particular food allergies. Such treatment should enable an individual suffering from an allergy to overcome his condition to such an extent that he can safely risk future exposures to allergens associated with his particular type of allergy. This would greatly benefit the lifestyle of the patient as he will no longer have to scrutinize his diet for the possible presence of allergens.

In accordance with the invention it has been found that dietary proteins can be modified in a specific manner that enable their use in immunotherapy. Accordingly, the invention relates to a method for treating an individual suffering from a food allergy or having a tendency to develop a food allergy by administering to said individual a modified food allergen, wherein the food allergen is modified by reduction and alkylation.

In broad terms, the method of the invention is aimed at treating individuals suffering from or having a tendency to develop a food allergy brought about by an allergenic food protein having one or more disulfide bonds. As such, the method may be practised in the form of a therapy, but also as a prophylactic treatment. The method comprises administering to an individual a therapeutically effective amount of a modified form of the allergenic food protein. The modification performed in accordance with the present invention results in reduced disulfide bond forming ability of the protein.

The primary amino acid sequence and positions of disulfide bonds strongly influences the overall structure of protein. For example, certain side-chains will permit, or promote, hydrogen-bonding between neighbouring amino acids of the polypeptide backbone resulting in secondary structures such as β-sheets or α-helices. These secondary structures can interact with other secondary structures within the same polypeptide to form motifs or domains (i.e. tertiary structure). A motif is a common combination of secondary structures and a domain is a portion of a protein that folds independently. Many proteins are composed of multiple subunits and therefore exhibit a quaternary structures.

Without wishing to be bound by theory it is believed that the modification of the allergenic food protein by reduction and alkylation results in breakage of disulfide bonds so as to result in a dithiol, followed by effective prevention of disulfide bonds again being formed due to the presence of the alkyl chains.

Surprisingly, exposure of an allergic individual to an allergen modified according to the invention is not only safe and does not cause any significant allergic reactions, it is also possible to effectively desensitize the individual to the allergen. Presenting the individual's immune system with an allergen modified in accordance with the invention has been found to lead to a reduction or prevention of the production of specific-IgE antibodies. In an individual with a developed allergy, the IgE response of the immune system may be down-regulated skewing the immune response from a Thelper-2 mediated reaction towards a Thelper-1 mediated reaction. As a result, after completion of the treatment, the individual no longer needs to avoid intake of foodstuffs containing the dietary protein to which he used to be allergic.

The term "allergen" is used here and subsequently to mean a substance which, when exposed to a mammal, will be able to mount an immune response resulting in antibodies of the IgE-class and which also will be able to trigger an allergic reaction when the mammal later on is exposed to the substance. Allergens in terms of the present invention are allergenic food proteins, which may consist of protein or a protein combined with a lipid or a carbohydrate such as a glycoprotein, a proteoglucan, a lipoprotein etc.

An allergen which is modified according to the invention for use in immunotherapy may in principle be any allergenic food protein. Typical allergenic food proteins often containing disulfide bonds or bridges (herein the terms "bonds" and "bridges" in the context of disulfide bridges will be used interchangeably), which are relatively stable to heat and to digestion (e.g. the effect of pepsin). In a preferred embodiment, the allergen belongs to the family of seed storage proteins of which 2S albumins, lipid transfer proteins (LTP's) and conglutins are particularly preferred. Of the 2S albumins, tree nut and seed 2S albumins, and in particular Brazil nut 2S albumin, are preferred. Other examples of tree nut 2S albumins are those from hazelnut, walnut, almond, cashew nut, pistachio nut, pecan nut, chest nut, macademia, nangai nut, acorn, and pine nut. Examples of seed 2S albumins are those from linseed, poppy seed, rape seed, sesame seed, and sunflower seed.

2S albumins are storage proteins in seeds and nuts that share common features regarding structural organization. They are small globular proteins composed of a small and large subunit that are held together by disulfide bonds. Both subunits originate from one gene, expressed as a single chain peptide that is proteolytically processed. For 2S albumin from Brazil nut *(Ber e 1)*, the large subunit is 9 kDa, and the small subunit is 3 kDa. Another common feature of 2S albumins is their amino acid composition. They are rich in arginine, glutamine, asparagine, and cysteine. In particular, Brazil nut 2S albumin has a high content of cysteine (8%) and methionine (19%) as well. Sulfur-containing amino acids are essential for the human and animal diet, and 2S albumin from Brazil nut is considered a valuable nutrient. The amino acid sequence of 2S albumins in different species is homologous and the cysteine residues are conserved. Within the species Brazil nut, several isoforms of 2S albumin have been found with heterogeneity in the light chain. In general, it has been found that the presence of disulfide bonds increases thermostability, is often encountered in proteins of thermophilic bacteria, increases conformational stability at ambient temperatures, and reduces the susceptibility for enzymatic digestion. Also, Brazil nut 2S albumin's structure exhibits a large stability towards heat denaturation and guanidinium induced unfolding.

Prior to modification, the allergen is preferably isolated from its biological source, such as the nut. It is, however, also possible to modify a crude extract comprising the allergen together with other components of the biological source. Although this may result in administration to a patient of other proteins or other substances modified by reduction and alkylation, this is not considered to be harmful. Therefore, the present invention pertains to administration of isolated and subsequently modified proteins as well as to crude extracts from allergen-containing food items, preferably nuts, such as obtainable by milling, grinding, etc. which have been subjected to reduction and alkylation reactions according to the present invention.

Isolation of the allergen may be done by any known method, such as methods involving extraction and liquid chromatography. Methods for isolating allergens from various biological sources are known per se and may be conveniently adapted to the needs of the circumstances by the skilled person based on his common general knowledge.

The allergen may also be obtained commercially, such as for instance from Allergen AB, Ängelholm, Sweden, from ALK Albello, Horsam, Denmark, or from Pharmacia Diagnostics AB, Uppsala, Sweden.

In accordance with the invention, the allergen is modified by reduction and alkylation. It is preferred that reduction is carried out prior to alkylation. Reduction and alkylation of proteins are known *per se.* For an overview, reference is made to Herbert *et al*., Electrophoresis (2001), 22:2046. It will be understood that it is preferred that only reagents are used which are acceptable in the context of the production of foodstuffs or pharmaceuticals.

In a preferred embodiment, reduction is performed using a reducing agent chosen from the group of 2-mercaptoethanol (β-ME), dithiothreitol (DTT), dithioerythritol, cysteine, homocystein, tributylphosphine, sulphite, sodium (cyano) borohydride, lye, glutathione, E-mercapto ethylamine, thioglycollic acid, methyl sulfide, ethyl sulfide and combinations thereof. In general, alkylthiol compounds (R-SH) provide suitable reducing agents. Preferably, those reducing agents are used that disrupt the disulfide bonds while maintaining other chemical characteristics of the protein. For instance, NH₂ groups are preferably left intact.

Alternatively, reduction may be performed by using enzymatic means, such as by using proteins that catalyse thiol-disulfide exchange reactions such as for instance glutaredoxin or thioredoxin. Such proteins may exert their effect via two vicinal (CXYC) cysteine residues, which either form a disulfide (oxidized form) or a dithiol (reduced form). Alternatively proteins may be used that are capable of catalysing the rearrangement of both intrachain and interchain-S-S-bonds in proteins such as protein disulfide isomerase or other polypeptides capable of reducing disulfide bonds such as for instance disclosed in WO 00/70064.

Preferably, the reduction reaction is continued until the reaction stops and essentially all disulfide bonds in the allergen have been broken.The conditions under which reduction is carried out can be optimised depending on the chosen reducing agent by the skilled person based on his general knowledge. Typically, reduction will be carried out at neutral, or near neutral pH, preferably at a pH between 6 and 8, at concentrations of reducing agents in the suitable range of, or equivalent to, for instance about 1-100 mM of DTT (or β-ME), possibly by using a suitable buffer. An example of a suitable buffer comprises chaotropic reagents, such as guanidine and/or urea. The temperature during reduction will generally lie between ambient or room temperature and 70°C, optionally under a reducing atmosphere, such as an anoxic atmosphere, preferably a nitrogen (N₂) atmosphere. Of course, care should be taken that the allergen does not denaturise during the reaction.

Alkylation is preferably carried out by blocking the SH-radicals that result from the cleavage of the disulfide bonds during reduction. Preferred alkylation reagents are chosen from the group of N-ethylmalimide, cystamine, iodoacetamide, iodoacetic acid. More generally, at least one disulfide bond can be reduced and alkylated to produce cysteine residues with side chains having the chemical formula -CH₂-S-[CH₂]ₙ-R' wherein n is an integer between 1 and 5 and R' is selected from the 1-5 carbon groups consisting of alkyl groups (e.g., methyl, ethyl, n-propyl, etc.); carboxy alkyl groups (e.g., carboxymethyl, carboxyethyl, etc.); cyano alkyl groups (e.g., cyanomethyl, cyanoethyl, etc.); alkoxycarbonyl alkyl groups (e.g., ethoxycarbonylmethyl, ethoxycarbonylethyl, etc.); carbomoylalkyl groups (e.g., carbamoylmethyl, etc.); and alkylamine groups (e.g., methylamine, ethylamine, etc.). Other suitable alkylating reagents include alkylhalogenides; alkylsulfates; alkenes, preferably terminal alkenes (H₂C)=C(H)-R); and other alkylating reagents known to one skilled in the art.

Alternatively, alkylation may be performed by using enzymatic means, such as by using sulfhydryl oxidase, for instance as may be obtained from chicken egg protein.

Although strictly not an alkylation reaction, the oxidation of the SH-radicals towards for instance SO₂ or SO₃ forms an aspect of the present invention since the reformation of the protein disulfide bonds is effectively blocked as a result thereof.

The conditions under which reduction is carried out can be optimised depending on the chosen reducing agent by the skilled person based on his general knowledge. Typically, alkylation will be carried out at neutral, or near neutral pH, preferably at a pH between 6 and 8, possibly be using a suitable buffer. The temperature during alkylation will generally lie between ambient or room temperature and 50°C.

The modified allergenic protein that is obtained after the reduction and alkylation reactions is characterized in having a considerably altered secondary structure. For instance, the β-sheets or β-turns encompassed in the native protein may be absent. It is also conceivable that the β-structures remain (partially) intact and that α-helices are absent. Tertiary structuring may also be reduced as evidenced by the free energy of the protein. In general, the digestability by peptidases of the modified protein is improved.

The above described modified allergen is, in accordance with the invention, used in immunotherapy. In this context, it may be administered in any suitable dosage form.

It will be understood that the invention also relates to a pharmaceutical composition comprising the modified allergen for immunotherapy directed against food allergy. A pharmaceutical composition of the invention comprises a therapeutically effective amount of the modified polypeptides of the claimed invention. Once formulated, the pharmaceutical compositions of the invention can be administered directly to the subject. Direct delivery of the compositions will generally be accomplished by injection, either orally, subcutaneously, sublingually, intraperitoneally, intravenously or intramuscularly, pulmonarily, or delivered to the interstitial space of a tissue.

The pharmaceutical composition may also comprise a suitable pharmaceutically acceptable carrier and may be in the form of a capsule, tablet, lozenge, dragee, pill, droplets, suppository, powder, spray, vaccine, ointment, paste, cream, inhalant, patch, aerosol, and the like. As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the modified allergen. It may be preferred to further include an adjuvant, preferably one known to skew the immune response towards a Thelper-1 mediated response, in the dosage form, in order to further stimulate or invoke a reaction of the patient's immune system upon administration. Suitable adjuvants include such adjuvants as complete and incomplete Freund's adjuvant and aluminium hydroxide. It is also conceived that the modified allergen is incorporated in a foodstuff and is administered to a patient together with food intake.

A pharmaceutical composition may also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

For therapeutic treatment, modified allergenic proteins may be produced as described above and applied to the subject in need thereof. The modified allergenic proteins may be administered to a subject by any suitable route, preferably in the form of a pharmaceutical composition adapted to such a route and in a dosage which is effective for the intended treatment. Therapeutically effective dosages of the modified allergenic proteins required for decreasing the allergenic reaction to the native form of the protein or for desensitising the subject can easily be determined by the skilled person, for instance by using animal models.

The term "therapeutically effective amount" as used herein refers to an amount of a therapeutic, viz. a modified allergenic food protein according to the present invention, to reduce or prevent allergic reactions to allergenic food proteins, or to exhibit a detectable therapeutic or preventative effect. The effect can be detected by, for example, reduced IgE antigen levels to the allergenic food protein. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the routine judgment of the clinician or experimenter. Specifically, the compositions of the present invention can be used to reduce or prevent allergic reactions to allergenic food proteins and/or accompanying biological or physical manifestations. Such manifestations may include contraction of smooth muscle in the airways or the intestines, the dilation of small blood vessels and the increase in their permeability to water and plasma proteins, the secretion of thick sticky mucus, and, in the skin, redness, swelling and the stimulation of nerve endings that results in itching or pain. Manifestations that may be prevented by immunotherapy according to the present invention include skin manifestations such as rashes, hives or eczema; gastrointestinal manifestations including cramping, nausea, vomiting or diarrhoea; or respiratory manifestations including sneezing or runny nose, coughing, wheezing or shortness of breath. Other manifestations that may be prevented may include be itching of skin, flushes, congestion, eye irritation, asthma, itching in the mouth or throat which may progress to swelling and anaphylaxis. Methods that permit the clinician to establish initial immunotherapy dosages are known in the art (e.g. US patent 4,243,651). The dosages determined to be administered must be safe and efficacious.

For purposes of the present invention, an effective dose will be from about 0.1 ng/kg to 0.1 mg/kg or 10 ng/kg to about 10 µg/kg of the modified allergenic food protein in the individual to which it is administered. These dosages are intended for modified allergens obtained from purified allergens. For modified allergens based on a crude extract of the allergen, dosages may be higher corresponding to the purity of the extract used.

For typical desensitization treatment, it is typically necessary for the patient to receive frequent administrations, e.g., initially every two or three days, gradually reducing to once every two or three weeks. Other suitable desensitisation programs include subcutaneous injections once every 2-4 weeks the dosage of which injections may gradually increase over a period of 3-6 months, and then continuing every 2-4 weeks for a period of up to about 5 years.

Desensitization protocols may also comprise a form of treatment conventionally known in various equivalent alternative forms as rapid desensitization, rapid allergen immunotherapy, rapid allergen vaccination, and rapid or rush immunotherapy. In broad terms, this procedure aims to advance an allergic patient to an immunizing or maintenance dose of extract (i.e., allergen) by administering a series of injections (or via another suitable carrier) of increasing doses of the allergen at frequent (e.g. hourly) intervals. If successful, the patient will exhibit an improved resistance to the allergen, possibly even presenting a total non-reactivity to any subsequent allergen exposure. Various desensitization protocols are known in the art and may for instance comprise a method of treating a patient having an immediate hypersensitivity to an allergen using an accelerated rapid immunotherapy schedule in combination with a method of pretreating such patient with prednisone and histamine antagonists prior to receiving the accelerated immunotherapy such as described in US patent application 2003/082212.

Yet in another alternative embodiment, the modified allergens or compositions of the invention may be administered from a controlled or sustained release matrix inserted in the body of the subject.

The invention will now be elucidated by the following, non-restrictive examples.

### EXAMPLES

### Materials and Methods

### Brazil nut protein extraction and liquid chromatography

Brazil nuts (*Bertholletia excelsa*) were purchased as unshelled nuts from Imko Nut Products (Doetinchem, The Netherlands) and were stored vacuum-sealed at 10°C until use. One kg Brazil nuts were ground and extracted three times with petroleum ether. A portion of 368 gram defatted meal was extracted with 3680 mL 20 mM sodium acetate, pH 5.5, for 2 hours at room temperature. The insoluble fraction was discarded after centrifugation (30 minutes at 8000g). The extract was stored in portions at -20°C.

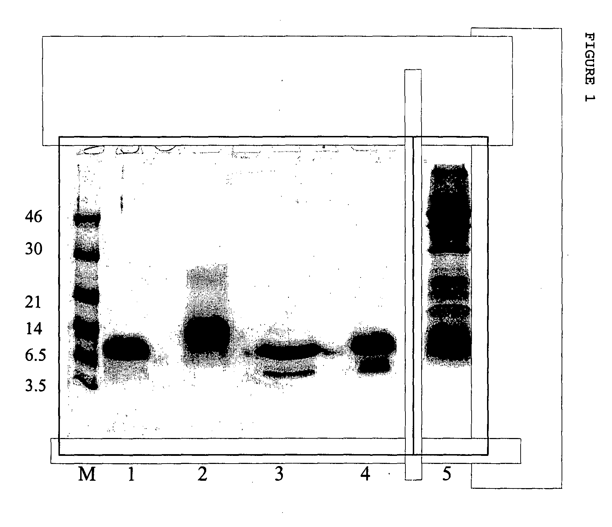

A fraction of 2 L was applied to a Source S column (300 mL column volume, Pharmacia & Upjohn, Uppsala, Sweden) equilibrated with 20 mM sodium acetate, pH 5.5 (loading buffer). After washing the column with loading buffer to remove unbound protein, the column was eluted with a 3 L gradient from 0 to 1 M sodium chloride in loading buffer. 2S albumin-containing fractions were pooled and stored in small portions for single use at -20°C. All buffers used were filtered over 0.45 µm membranes. Under non-reducing conditions, purified 2S albumin migrates as a single band with an estimated purity of >95% at approximately 12 kDa on SDS-PAGE (Figure 1). Purified 2S albumin was dialyzed against demineralized water, lyophilized and stored at -20 °C.

### Protein determination

Protein concentrations in crude extracts were measured using the Bradford method (BioRad, Hercules, CA) with bovine serum albumin as a standard. Concentrations of purified 2S albumin were determined by measuring the A280. A value for A280 (1 mg/ml) of 0.125 was used, based on the Web-based program ProtParam from SwissProt (www.expasy.org/tools/protparam.html, sequence code AB044391).

### Reduction and alkylation of 2S albumin

Lyophilized protein (540 mg) was dissolved in 6 M guanidinium, 100 mM NH₄HCO₃ pH 7.8 (270 mL; 2 mg/mL) and then the solution was warmed to 56 °C and dithiotreitol (DTT) (830 mg; 20 mM final concentration) was added. After 60 min. the reduced protein was allowed to cool to room temperature and a freshly prepared iodoacetamide solution (5.02 g in 27 mL 100 mM NH₄HCO₃ pH 7.8; 100 mM final concentration) was added to the stirred protein solution. The alkylation was allowed to proceed for 90 min. at room temperature in the dark. The reduced and alkylated protein was dialyzed against demineralized water (3 times 10 L) in Spectro/Por 6 (1 kDa cut off) dialysis tubing at 4 °C. As control 2S albumin (110 mg) was dissolved in 6 M guanidinium, 100 mM NH₄HCO₃ pH 7.8 (50 mL; 2 mg/mL), was heated to 56°C, cooled to room temperature and then dialyzed.

Both the alkylated 2S albumin and the control were lyophilized. The akylated 2S albumin (524 mg, yielding 97%) was obtained as a crystalline slightly yellow dense powder and the control 25 albumin (110 mg, 100%) was obtained as a white fluffy powder. Both the alkylated and control 2S albumin were dissolved (2 mg/mL) in 100 mM NH₄HCO₃ pH 7.8. Four hundred µL protein solution and 100 µL DTNB solution (5,5'-dithiobis(2-nitrobenzoic acid) or Ellman's reagent, 50 mM in NH₄HCO₃ pH 7.8) were mixed and the absorption at 405 nm was measured after 10 min incubation at room temperature. As a blank 400 µL buffer and 100 µL DTNB solution were mixed. The number of free ―SH groups was calculated using the molar extinction coefficient (A405 = 13260 M-1 cm-1) of the free 5-thio2-nitrobenzoic acid (TNBS) anion. Reduced and alkylated 2S albumin showed 0.01 free ―SH groups per molecule, and in the control preparation, no free ―SH groups could be detected. The reduced and alkylated 2S albumin shows a large and small subunit, at approximately 9 and 3 kDa, respectively (Figure 1). This preparation is further denoted as RA-2S albumin.

The heavy and light chains of RA-2S albumin were isolated using size exclusion chromatography. The reduced, alkylated and lyophilized 2S albumin was dissolved in 20 mM sodium phosphate buffer containing 100 mM NaCl. The heavy and light chain were separated using an Äkta Explorer FPLC (Amersham Pharmacia Biotech) equipped with a SD-30 peptide gel filtration column (475 mL, Amersham-Pharmacia Biotech), calibrated with a mixture of globular proteins and peptides of known mass according to the instruction of the column manufacturer. Separation was performed batch-wise (4 mL,10 mg/mL portions) with a flow rate of 1 mL/min and proteins were monitored at 214 nm.

### Animals

For the sensitization studies young male Brown Norway (BN) rats (3-4 weeks old at arrival) obtained from Charles River (Sulzfeld, Germany) were used throughout these studies. During the experiment and for at least 10 days prior to study initiation the rats were housed in an animal room maintained at 23 ± 3°C, with a light/dark cycle of 12 h, and a relative humidity of 30-70%. The animals were housed in macrolon cages in groups of five and had access to food and tap-water ad libitum. As the major allergen (*Ber e1*) from Brazil nut was used for sensitization studies, the rats were bred and raised on a commercially available Brazil nut free rodent diet (SDS Special Diet Service, LAD1 (E) SQC, Witham, England). To ensure the use of immunologically naive animals with respect to the antigens under investigation pre-study blood samples were always tested for Brazil nut specific antibodies. All animal studies were approved by an independent ethical committee.

### Sensitization protocol

For the oral sensitization animals (n=10) were exposed by gavage dosing using a 18-gauge stainless steel animal feeding needle during 6 weeks. Rats received either reduced/alkylated 2S albumin (RA-2S albumin) or native 2S albumin (0.1 mg or 1 mg protein/ml tap-water; 1 ml/animal) without the use of an adjuvant or only water (controls). Blood samples were obtained from the orbital plexus under light CO₂ anaesthesia at day 0 or by exsanguination from the abdominal aorta at sacrifice. After coagulation for 1 h at room temperature, the blood samples were centrifuged (Heraeus Minifuge T, Osterode, Germany) for 20 min. at 2000g and 4°C to obtain sera. The sera were stored in aliquots at -20°C until analyses for anti-native- and RA-2S albumin specific IgG1 and IgG2a titers by Enzyme Linked Immunosorbent Assay (ELISA) and specific IgE by passive cutaneous anaphylaxis (PCA)-test.

Positive control animals (n=5) were injected intraperitoneally (i.p.) with 0.5 ml of a 0.2 mg/ml reduced and alkylated or native 2S albumin solution in sterile saline on days 0, 2, 4, 7, 9 and 11. To potentiate the immune response, 0.2 ml of a 25 mg/ml Al(OH)₃ adjuvant suspension in sterile saline mixed with the 0.5 ml of (RA)-2S albumin was injected at day 0. The animals were bled on day 28 by exsanguination from the abdominal aorta. Sera were prepared and stored as described above.

### Measurement of serum specific IgG1 and IgG2a antibodies

ELISA-techniques were used to measure serum antibodies specific for native 2S albumin and RA-2S albumin. For the detection of RA-2S albumin or native 2S albumin-specific IgG1 or IgG2a, 96-wells microtiter plates (Flat-bottomed, Maxisorp, NUNC, Roskilde, Denmark) were coated overnight at 4°C with 100 µl/well of a 5 µg/ml solution of RA-2S albumin or native 2S albumin in carbonate buffer, pH 9.6. The plates were washed three times with tap-water containing 0.4% Tween 20 (Merck, Hohenbrunn, Germany). This was followed by the addition of 100 µl/well phosphate-buffered saline (PBS) containing 1% bovine serum albumin (BSA; Sigma Chemicals Co., St. Louis, USA) and 0.02% Tween 20 (PBS/BSA-Tween 20). After 1 h incubation at 37°C, the plates were washed and serial dilutions of rat serum in PBS/BSA-Tween 20 were added to the wells and incubated for 1 h at 37°C. After washing, 100 µl/well peroxidase conjugated mouse anti-rat IgG1 or IgG2a (Zymed, San Francisco, USA, diluted 1:500) in PBS/BSA-Tween 20 was added. After incubation for 1 h at 37°C, the plates were washed again and an enzyme substrate solution of 3,3',5,5'-tetramethylbenzidine (TMB; Sigma Chemicals Co., St. Louis, USA, 100 µl/well; 6 mg/ml DMSO) was added. The plates were developed at room temperature for 5 to 15 min. Finally, 100 µl/well of 2N H₂SO₄ was added. Optical densities were read spectrophotometrically at 450 nm with an ELISA plate reader (Microplate Reader, Bio-rad Laboratories, Richmond, USA). A presera pool was used as negative control. The pooled preserum was measured at a 1:4 dilution. The average extinction in negative control wells, to which three times the standard deviation was added, provided the reference value taken to determine the titer in the test sera. Each test serum was titrated starting at a 1:4 dilution and the reciprocal of the furthest serum dilution giving an extinction higher than the reference value was read as the titer. All analyses were performed in duplicate. Positive and negative control samples were incorporated for each 96-wells plate.

### Measurement of serum specific IgE antibodies

Rat sera were tested for (RA)-2S albumin-specific IgE by ELISA. For the detection of (RA)-2S albumin-specific IgE, 96-wells microtiter plates were coated overnight at 4°C with 100 µl/well mouse anti-rat IgE (MARE-1, Zymed, San Francisco, USA) at a concentration of 1.5 µg/ml in carbonate buffer, pH 9.6. The plates were washed and 100 µl/well of PBS/BSA-Tween 20 was added. After incubation for 1 hour at 37°C, the plates were washed and diluted rat serum samples were added and incubated for 2 hours at 37°C. The plates were washed and subsequently, 100 µl/well of a 1 µg/ml solution of a (RA)-2S albumin-digoxigenin (DIG) conjugate was added. The DIG was obtained from Boehringer (Mannheim, Germany) and a coupling to (RA)-2S albumin was performed according to the manufacturer's instructions. The labelled (RA)-2S albumin was separated on a sephadex G-25 column (Boehringer, Mannheim, Germany) and labelling efficiency was determined spectrophotometrically at 280 nm. Incubation with (RA)-2S albumin-DIG was performed for 1 hour at 37°C. After washing, 100 µl/well peroxidase conjugated sheep anti-DIG Fab fragments (Boehringer, Mannheim, Germany) diluted 1:3000 in PBS/BSA-Tween 20 was added. After incubation for 1 hour at 37°C, the plates were washed again and an enzyme substrate solution of TMB was added. Plate development, measurement and titer elaboration were as described for the (RA)-2S albumin-specific IgG1/IgG2a ELISA.

### Measurement of reagenic antibodies by Passive Cutaneous

### Anaphylaxis (PCA)

For PCA-testing the method as described previously by Ovary *et al*., Immunol. Methods (1964), 259-83 was used. Naive (untreated) BN rats were shaven on the back and flanks and injected intradermally with 0.1 ml of the test sera in serial dilutions, followed 64 h later with an intravenous injection of 1 ml of a 1:1 mixture of a solution of RA-2S albumin or native 2S albumin (5 mg/ml sterile saline) and a solution of Evans blue (2% in sterile saline). After 20-30 min., the animals were examined for positive responses. The diameter of dye extravasation at the site of the serum injection was measured. The reaginic titer was read as the reciprocal of the furthest dilution giving a colored spot of at least 5 mm in diameter. Positive and negative control sera as used in the ELISA's were assayed simultaneously with the test sera on each animal used for the PCA tests.

### Results

### Legends to figures

### FIGURE 1.

SDS page of 2S albumin and RA-2S albumin.

Lane 1: Native 2S albumin; lane 2: RA-2S albumin. Lane M shows marker proteins with known molecular weigh (shown in left margin, kDa).

### FIGURE 2.

2S albumin and reduced/alkylated 2S albumin (RA-2S albumin)-specific IgG1 (□) and IgG2a (■) titres upon daily intra-gastric dosing of BN rats (n=10) with water, 0.1 mg, and 1 mg 2S albumin, 0.1 mg and 1 mg RA-2S albumin per rat for 28 (Figure 1A) or 42 days (Figure 1B), respectively. Several animals (n=5) were sensitised intraperitoneally with either 2S albumin or RA-2S albumin together with alum as an adjuvant (Figure 1A). The data are presented as mean ²log Ig titre ± SD of the number of responding rats per group. The percentage of animals developing an IgG1 or IgG2a response at the respective time-points is indicated in the bars.

### FIGURE 3.

2S albumin and reduced/alkylated 2S albumin (RA-2S albumin)-specific IgE titres upon daily intra-gastric dosing of BN rats (n=10) with water, 0.1 mg, and 1 mg 2S albumin, 0.1 mg and 1 mg RA-2S albumin per rat for 42 days or after parenteral sensitisation with alum as an adjuvant (n=5) with either 2S albumin or RA-2S albumin (day 28 sera), respectively. The data are presented as mean ²log Ig titre ± SD of the number of responding rats per group. The percentage of animals developing an IgE response at the respective time-points is indicated in the bars.

### FIGURE 4.

2S albumin and RA-2S albumin-specific IgE as measured by passive cutaneous anaphylaxis tests upon daily intra-gastric dosing of BN rats (n=10) with water, 0.1 mg and 1 mg 2S albumin, 0.1 mg and 1 mg RA-2S albumin per rat for 42 days, respectively. Several animals (n=5) were sensitised intraperitoneally with either 2S albumin or RA-2S albumin together with alum as an adjuvant. Serum samples were from the last day of gavage dosing or 28 days after i.p. dosing. Symbols (□) indicate individual rats. Data are given as the individual measured wheal diameter in cm.

### Serum specific IgG1 and IgG2a levels

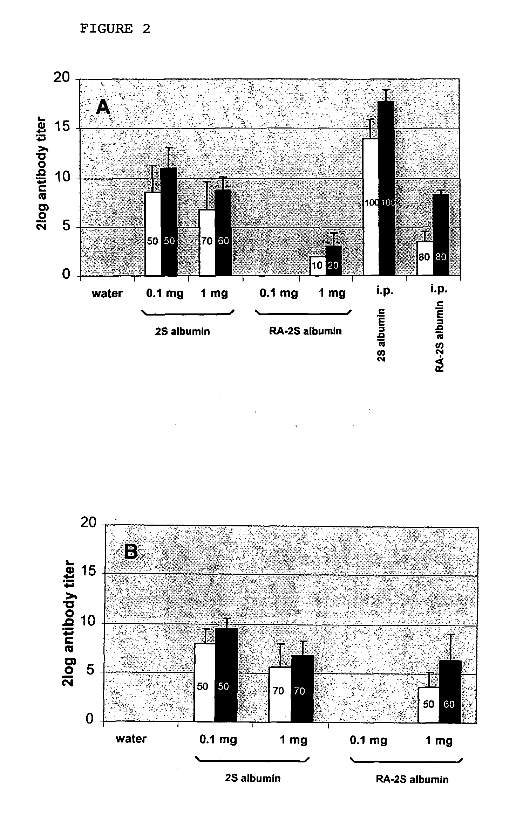

The serum levels of RA-2S albumin or native 2S albumin-specific IgG1 and IgG2a antibody levels were determined in days 28 and 42 sera (or on day 28 only in case of parenteral sensitization) using ELISA-technique (Figure 2A+B). Pre-study blood samples were negative for all animals. These sera were pooled and used as negative control in the ELISA's and PCA test. In general 70% of the animals orally exposed to 1 mg native 2S albumin/rat/day, developed native 2S albumin-specific IgG1 and IgG2a responses at days 28 and 42 whereas oral exposure to 1 mg RA-2S albumin resulted in a lower number of responding animals 10 and 20% at day 28 and 50 and 60% at day 42 for RA-2S albumin-specific IgG1 and IgG2a, respectively. Dosing with a lower amount of protein resulted in even more striking differences. Oral exposure of the animals to 0.1 mg native 2S albumin resulted in native 2S albumin specific IgG1 and IgG2a responses in 50% of the animals whereas no specific IgG1 or IgG2a responses were observed in the animals orally dosed with 0.1 mg RA-2S albumin at both days 28 and 42.

At day 28, parenteral exposure of the animals to native 2S albumin resulted in the development of very pronounced 2S albumin-specific IgG1 and IgG2a responses in all animals. Parenteral exposure to RA-2S albumin resulted in RA-2S albumin-specific IgG1 and IgG2a responses in 80% of the animals.

### Serum specific IgE levels

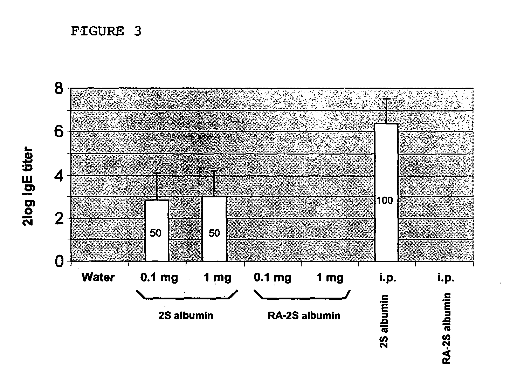

The serum levels of RA-2S albumin or native 2S albumin-specific IgE levels were determined in days 28 (parenteral sensitization) and 42 sera (oral sensitization) using ELISA-technique (Figure 3). Pre-study blood samples were negative for all animals. These sera were pooled and used as negative control in the ELISA's and PCA test. As shown in figure 3, both oral (0.1 or 1 mg protein/rat/day) and parenteral exposure of the animals to RA-2S albumin did not result in the development of RA-2S albumin-specific IgE antibodies. In contrast, oral dosing of the animals to either 0.1 mg or 1 mg native 2S albumin resulted in 2S albumin-specific IgE responses in 50% of the animals. Parenteral exposure to native 2S albumin induced 2S albumin-specific IgE responses in all animals tested.

### Reaginic antibody levels

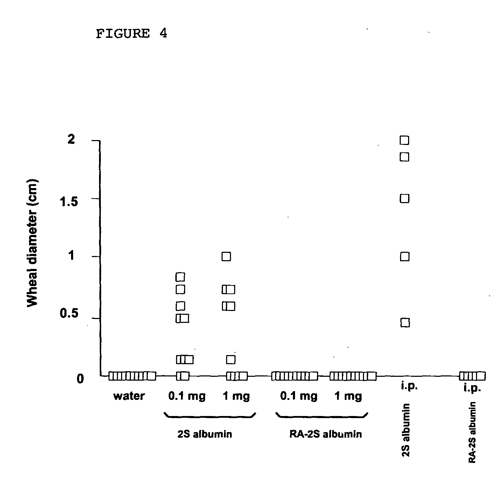

The presence of RA-2S albumin and native 2S albumin-specific reaginic antibody levels was determined in undiluted day 42 sera, or day 28 in case of parenteral sensitization, using passive cutaneous anaphylaxis tests. As shown in figure 4, both oral (0.1 or 1 mg protein/rat/day) and parenteral exposure of the animals to RA-2S albumin did not result in the development of RA-2S albumin-specific reaginic antibodies. In contrast, oral dosing of the animals to either 0.1 mg or 1 mg native 2S albumin resulted in 2S albumin-specific reaginic antibody responses in 50% of the animals. Parenteral exposure to native 2S albumin induced 2S albumin-specific reaginic antibody responses in all animals tested.

### Discussion

The present results show that reduction of the disulfide bonds of Brazil nut 2S albumin decreases the sensitizing potential of 2S albumin in the Brown Norway rat food allergy model. Oral exposure to RA-2S albumin did not result in the development of specific IgE against RA-2S albumin in any of the exposed animals indicating a loss of allergenicity of RA-2S albumin as determined both by ELISA and PCA. Oral administration of native 2S albumin resulted in the development of Thelper-1 mediated 2S albumin- specific IgG1 and Thelper-2 mediated 2S albumin-specific IgG2a and IgE responses in the rat. The allergenicity of RA-2S albumin was decreased but the immunogenicity of the protein still existed, although a lower level compared to native 2S albumin, since specific IgG1 and IgG2a antibodies against RA-2S albumin were formed.

Dosing of the animals with the low dose RA-2S albumin (0.1 mg protein/rat/day) did not result in an antibody response at all in the rats whereas the same dose of native 2S albumin induced specific IgG1, IgG2a and IgE responses, again indicating a lesser immunogenicity. Dosing of 1 mg RA-2S albumin resulted in specific IgG1 and IgG2a responses in the rats although the onset of the response was delayed compared to the animals treated with 1 mg native 2S albumin. Increasing the dose of RA-2S albumin might have resulted in allergic sensitization of the animals since it is known that several aspects determine the possible allergenicity of a protein in animal models, including age of first exposure, the route of exposure, duration and magnitude of exposure.

Taken together, these data show that reduction of the disulphide bonds of 2S albumin results in loss of allergenicity and an increased sensitivity to digestion.

## Claims

1. A method for treating an individual suffering from or having a tendency to develop a food allergy comprising administering to said individual a therapeutically effective amount of a modified food allergen, wherein the food allergen is modified by reduction and alkylation.

2. A method according to claim 1, wherein the food allergen is an allergenic food protein.

3. A method according to claim 1 or 2, wherein the food allergen is a seed storage protein.

4. A method according to claim 3, wherein the storage protein is a 2S albumin, lipid transfer protein (LTP), or conglutin.

5. A method according to claim 4, wherein the storage protein is a tree nut or seed 2S albumin.

6. A method according to claim 5, wherein the storage protein is brazil nut 2S albumin.

7. A method according to any of the preceding claims, wherein the food allergen is modified by reduction using a reducing agent chosen from the group of 2-mercaptoethanol, dithiothreitol, dithioerythritol, tributylphosphine, and a combination thereof.

8. A method according to any of the preceding claims, wherein the food allergen is modified by alkylation using an alkylating agent chosen from the group of N-ethylmalimide, cystamine, iodoacetamide, iodoacetic acid and combinations thereof.

9. A method according to any of the preceding claims, wherein the modified food allergen is administered in the form of a dosage form chosen from the group of a capsule, tablet, lozenge, dragee, pill, droplets, suppository, aerosol, powder, spray, vaccine, ointment, paste, cream, inhalant, or patch.

10. A method according to claim 9, wherein the dosage form further comprises a pharmaceutically acceptable carrier and/or an adjuvant.

11. A method according to any of the preceding claims, wherein the modified food allergen is administered orally, intraperitoneally, subcutaneously, intravenously, intramuscularly, pulmonarily, or via mucosa.

12. A method according to any of the preceding claims, wherein the modified food allergen induces production of Thelper-1 mediated subclasses of IgG antibodies.

13. A method according to any of the preceding claims, wherein the production of IgE antibodies is down-regulated.

14. A food allergen modified by reduction and alkylation for use in a method according to any of the preceding claims.

15. A pharmaceutical composition comprising an food allergen modified by reduction and alkylation for use in a method according to any of the claims 1-13.

16. Use of a food allergen modified by reduction and alkylation for the manufacture of a medicament for immunotherapy treatment of a food allergy brought about by the native form of said allergenic protein.
